# EUROPEAN PATENT APPLICATION

(11) **EP 4 625 360 A1**
(43) Date of publication of application: **01.10.2025**
(21) Application number: 24167699.8
(22) Date of filing: 28.03.2024
(51) Int. Cl.: G06V 20/52, G06V 10/98, G06V 10/25, H04N 7/18

(54) **INFANT MONITORING**

(71) Applicant: Koninklijke Philips N.V., 5656 AG Eindhoven (NL)
(72) Inventor: GOSENSHUIS, Daan Hendrik, Eindhoven (NL); BERNTSEN, Luc, Eindhoven (NL); SINGH, Jetendra Kumar, Eindhoven (NL); KANG, I-Chih, Eindhoven (NL); ASSELMAN, Michel Jozef Agnes, Eindhoven (NL)
(74) Representative: Philips Intellectual Property & Standards

(57) **Abstract**

Proposed concepts pertain to assessing if an image captured by an image sensor of a monitored area is suitable for use in image-based monitoring of an infant. In particular, embodiments aim to provide a method for assessing the suitability of an image based on determining a measure of the size and/or orientation of the region of the image that is to be used for infant monitoring. If the image is determined to be unsuitable, instructions are provided for adjustments to be made to the image sensor.

## Description

### FIELD OF THE INVENTION

This invention relates to the field of infant monitoring, and in particular to image-based infant sleep monitoring.

### BACKGROUND OF THE INVENTION

Image-based infant monitoring systems are often used by parents and carers to monitor an infant whilst they sleep, allowing the parent/carer to check in on the sleeping infant remotely. Infant monitoring systems are often provided with one or more sensors to take visual and/or audio data of a monitored area which may then be relayed to the parent via a separate device or via an app on the parent's smart phone or other personal smart device.

Modern infant monitoring systems may be provided with additional functionalities to track and monitor the behaviour of an infant while they sleep. These may include image-based monitoring of the breathing or vital signs of the infant or tracking the infant's movement. If the images obtained by the monitoring system are of poor quality, the results of these sleep monitoring processes may be unreliable.

### SUMMARY OF THE INVENTION

The invention is defined by the claims.

According to an aspect of the invention, there is provided a computer-implemented method for assessing the suitability of an image, captured by an image sensor, of a monitored area for use in monitoring an infant, the method comprising: determining a boundary of a target region within the image, or receiving an indication of a boundary of a target region within the image, wherein the target region is a region containing image data to be used for monitoring the infant; determining a measure of at least one of: a size; and an orientation of the target region of the image; determining, from the determined measure of the size and/or orientation of the target region, a suitability of the image for use in monitoring the infant; and providing an output based on determined suitability of the image.

Proposed concepts thus aim to provide schemes, solutions, concepts, designs, methods, and systems pertaining to assessing if an image captured by an image sensor of a monitored area is suitable for use in image-based monitoring an infant. In particular, embodiments aim to provide a method for assessing the suitability of an image based on determining a measure of the size and/or orientation of the region of the image that is to be used for infant monitoring. The image sensor may be any image sensor suitable for imaging an infant, including a sensor for optical imaging, ultrasound imaging, radar imaging, or lidar imaging. In some embodiments if the image is determined to be unsuitable, instructions are provided for adjustments to be made to the image sensor.

The infant monitoring preferably takes place during infant sleep, and it may provide information about the infant's sleep (such as periods of sleep and periods when awake). However, the same approach may be used more generally for image-based vital signs monitoring or other image-based monitoring functions, for example whether the infant is awake or asleep, or in light or deep sleep.

The invention will be described below, simply as one preferred implementation of the invention, with reference to sleep monitoring.

It is proposed to use the size and/or orientation of the region of an image that is to be used for monitoring an infant as a metric of the suitability of the image for this purpose. On the basis of this assessment, adjustments to the set-up of the image sensor used to capture the image may be suggested to ensure future images are of sufficient quality to permit accurate infant monitoring.

Modern image-based infant monitoring system may provide various functionalities for tracking and monitoring the behaviour of an infant, such as a sleeping infant. These rely on processing image data of the infant or their bed/crib to extract information regarding the infant, for example the presence, movement and vital signs of the infant. This may be achieved by processing the image data with a variety of different models and algorithms including motion detection models, pixel shift algorithms, object detection models, and edge detections models. In order that the output of these processing techniques is accurate, images must be of sufficient quality. For example, infant breathing monitoring relies on the accurate detection of very small motions of the infant's chest or upper torso and therefore requires the infant to be depicted with sufficient resolution that these motions can be detected.

It was realised that one way of ensuring accurate image-based monitoring of an infant, such as a sleeping infant, is to crop image data taken of a monitored area to include only a region of interest that contains the image data to be used for tracking the infant. For example, an image of an infant's crib may be cropped to only include the mattress area where the infant is likely to be found. This may reduce the noise produced by the background of the image and reduce processing requirements. The proposed method leverages determining the boundary of this cropped region and assessing the suitability of the image from a measure of the size or orientation of this region. The inventors have found that the size of the target region, (i.e., the region of the captured image that contains image data to be used in infant sleep monitoring) is a useful metric for assessing whether or not an image is of sufficient quality to allow for reliable infant monitoring. Likewise, the orientation of the target region (for example whether it is landscape or portrait) may also be a good indicator of image quality as it may indicate the position of the image sensor relative to the sleeping infant.

Ultimately, an improved computer-implemented method for assessing the suitability of an image, captured by an image sensor, of a monitored area for use in assessing the sleep of an infant may be supported by the proposed concept(s).

In some embodiments, the method determines a measure of the size of the target region, and determining a suitability of the image for use in monitoring the infant may further comprise: determining the image to be suitable if the measure of the size of the target region is greater than a predetermined threshold size; and determining the image to be unsuitable if the measure of the size of the target region is less than the predetermined threshold size. Thus, the method may allow for a simple binary determination of whether the image is suitable or not based on a predetermined threshold size. This may allow for known factors about the required quality of an image to be incorporated into the suitability assessment step.

In some embodiments, the measure of the size of the target region may comprise the area of the target region in pixels and the predetermined threshold size is between 550,000 and 950,000 pixels. Target regions with areas greater than such a threshold value have been shown to permit accurate monitoring of a sleeping infant including breathing monitoring and movement tracking.

In some embodiments, the measure of the size of the target region may comprise at least one of: an area of the target region; a dimension of the target region; and the proportion of the image covered by the target region. Thus, any suitable measurement of the target region may be used as a measure of its size. Different measures may be useful for different infant monitoring functions.

In some embodiments, the measure of the orientation of the target region may comprise at least one of: portrait; landscape; and an angle between an axis of the target region and an axis of the image. The orientation of the target regions may be a useful indicator of the horizontal distance between the image sensor and the infant being monitored and therefore may be a useful metric of whether the image is suitable for infant monitoring.

In some embodiments, determining a boundary of a target region within the image may comprise processing the image with at least one of: an object detection model; an edge detection model; and a contrast enhancement model. This allows for the process of determining the target region in the image to be entirely automated. For example, in the case where the monitored area corresponds to the infant's crib, a machine learning model may be trained to detect the area of an image that depicts the mattress of the crib and output the bounding box associated with this detection.

In some embodiments, receiving an indication of a boundary of a target region within the image comprises receiving data provided by a user using a user interface. This allows for the user to select the area that corresponds to the target region. This may be more flexible and potentially more reliable than automatic selection of the target region.

In some embodiments, providing an output based on the determined suitability of the image comprises providing instructions for adjusting the image sensor when the image is determined not to be suitable. In this way, the suitability assessment may be used directly provide suggestions for improving the image sensor set-up to ensure future images are of sufficient quality for infant monitoring.

In some embodiments, providing instructions for adjusting the image sensor when the image is determined not to be suitable comprises at least one of: providing an instruction to adjust the position of the image sensor; providing an instruction to adjust the orientation of the image sensor; providing an instruction to adjust the operating parameters of the image sensor; and providing an instruction to adjust the optical zoom of the image sensor. Thus, various parameters related to the image sensor set-up may be adjusted to improve the quality and hence suitability of captured images. Different adjustment instructions may be provided depending on the specific configuration of the image sensor.

In some embodiments, monitoring the infant may comprise at least one of: monitoring the infant's breathing; monitoring the infant's vital signs; monitoring the infant's movement; and tracking the infant's sleep stages. Image-based monitoring of a sleeping infant may track various behaviours of the infant to gather information about the quantity and quality of sleep the infant is getting. These different monitoring functionalities may have different requirements on the quality of the image of the monitored area. Therefore, the suitability assessment method of the present invention may be tailored depending on the specific subsequent processing steps intending to be carried out on the image.

According to another aspect of the present invention, there is provided a computer program comprising code means for implementing any of the methods described above when said program is run on a programming system.

According to yet another aspect of the invention, there is provided a processing arrangement comprising the computer program and configured to execute the computer program.

There is further provided a system for assessing the suitability of an image, captured by an image sensor, of a monitored area for use in monitoring an infant, the system comprising: a processing arrangement configured to execute the above computer program.

In some embodiments, an infant monitoring system may further comprise an image sensor configured to capture the image of the monitored area.

In some embodiments, the infant monitoring system may further comprise at least one of: a user input interface configured to permit the user to select the boundary of the target region within the image; and output interface configured to: provide to the user an indication of the suitability of the image for use in monitoring the infant; and provide instructions to the user for adjusting the image sensor when the image is determined not to be suitable.

In some embodiments, the processing arrangement of the infant monitoring system may be configured to provide instructions to automatically adjust the operating parameters of the image sensor. Thus, the system may be equipped with the ability to automatically adjust the set-up of the image sensor to ensure suitable images are captured, removing the need for user input and thereby reducing instances of user produced errors to the image sensor arrangement.

Embodiments may be employed in combination with conventional/existing infant monitoring methods and systems. In this way, embodiments may integrate into legacy systems to improve and/or extend their functionality and capabilities. An improved infant monitoring system may therefore be provided by proposed embodiments.

Thus, there may be proposed concepts for assessing the suitability of an image for use in monitoring an infant by determining a measure of the size and/or orientation of a target region within the image. These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figs. 1A and 1B depict simplified flow diagrams of methods for assessing the suitability of an image, captured by an image sensor, of a monitored area for use in monitoring the an infant according to two proposed embodiments;
Fig. 2 illustrates an image containing a target region to be used for monitoring an infant according to a proposed embodiment;
Fig. 3 depicts an infant monitoring system set-up according to a proposed embodiment;
Fig. 4 depicts a simplified block diagram of an infant monitoring system according to a proposed embodiment; and
Fig. 5 illustrates a simplified block diagram of an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes, and/or solutions pertaining to assessing the suitability of an image for use in monitoring an infant (for example, for use in image-based monitoring of the movement or vital signs of an infant for example when sleeping, or for use in image-based tracking of infant sleep periods). According to proposed concepts, several possible solutions may be implemented separately or jointly. That is, although these possible solutions may be described below separately, two or more of these possible solutions may be implemented in one combination or another.

Embodiments of the invention aim to provide a concept for automatically assessing whether an image is suitable for monitoring the infant present in the monitored area. In particular, a measure of the size and/or orientation of a certain target region of the image to be used for infant monitoring is used as an indicator of the quality of the image. The suitability of the image is then determined from these measures.

Modern infant monitoring systems are equipped with advanced infant sleep tracking functionalities. These may include processing image data of a monitored area with a model or algorithm to extract information about whether an infant is present in the monitored area, the infant's pose, the infant's activity level and the infant's vital signs. For this process to be reliable the infant and the monitored area must be imaged with sufficient resolution. The size and/or orientation of a target region of the image data that is to be used for infant sleep tracking is a useful indicator of the relative position of the image sensor and the infant's sleeping position and therefore can be used to provide an accurate assessment of whether an image is suitable to be used for advanced infant monitoring.

If an image is found to be unsuitable, the proposed system may further provide instructions for adjusting the image sensor to improve the suitability of future images captured by the sensor. Therefore, the proposed method may aid a process of setting-up an infant monitoring system for accurate monitoring.

By way of summary, an exemplary embodiment comprises the following modules:
A. Target Region Identification: the region of the image data that is to be used for monitoring the infant is identified and its boundary determined. This may be achieved automatically or via user inputted data.
B. Size/Orientation Determination: a measure of the size and/or orientation of the target region is determined. This measure may include the area or dimensions of the target area or the proportion of the total image that the target region covers and an indication of whether the target region is portrait or landscape.
C. Suitability Assessment: using the determined measure of the size and/or orientation of the target region a predetermined suitability logic is used to determine whether or not the captured image is suitable for use.
D. Output Instructions: instructions pertaining to whether or not the captured image is suitable for use, and any adjustments that could be made to the infant monitoring set-up to improve this suitability are output to a user or external device or system.

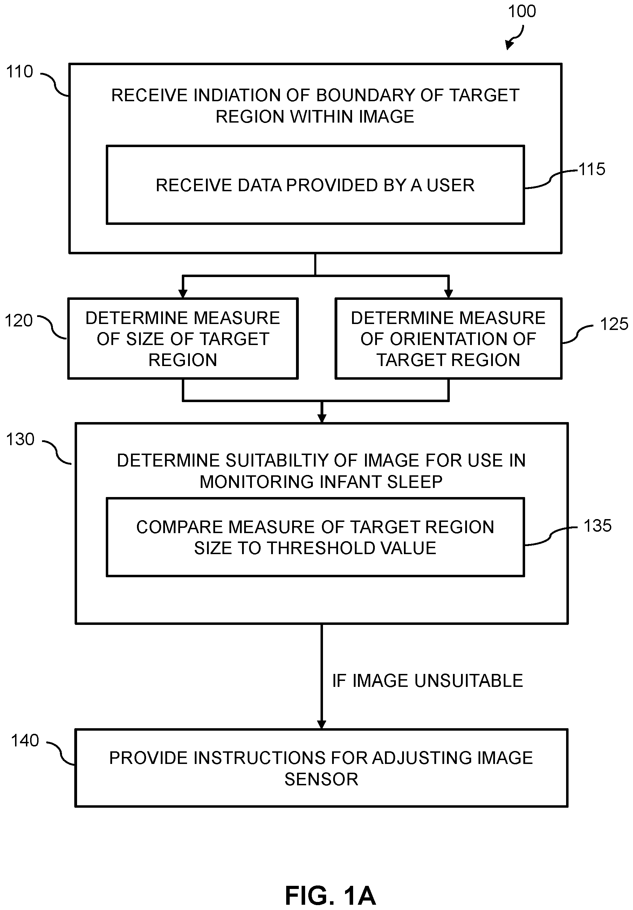

Referring now to Fig. 1A, there is depicted a simplified flow diagram of a computer-implemented method 100 for assessing the suitability of an image for use in monitoring an infant, for example for monitoring the sleep of an infant.

The method will be explained with reference to the preferred implementation according to which an infant is monitored during sleep.

The method commences with a step 110 comprising receiving an indication of a boundary of a target region within the image, wherein the target region is a region containing image data to be used for monitoring the sleep of the infant. In this exemplary embodiment, the step 110 further comprises the sub-step 115 comprising receiving data provided by a user using a user interface. For example, the image may be presented to the user via a user interface and the user asked to select which region of the image corresponds to the target region. In this example the image depicts the crib of an infant and the user is asked to select the region of the image that depicts the mattress area of the crib.

Once the boundary of the target area is received, the method proceeds to a step 120 of determining a measure of the size of the target region. In this exemplary embodiment, this comprises determining the total number of pixels of the image that are within the boundary of the target region.

In a step 125, a measure of the orientation of the target region is also determined. In this exemplary embodiment, the target region is rectangular and determining the orientation of the target region comprises determining whether the target region is in a portrait or landscape orientation. This is achieved by comparing the width of the target region to the height of the target region.

Once measures of the size and orientation of the target region have been determined, the method proceeds to a step 130 of determining, from the determined measures of the size and orientation of the target region, a suitability of the image for use in monitoring the sleep of the infant. In this exemplary embodiment, the step 130 comprises the sub-step 135 of comparing the measure of the size of the target region to a threshold value. The determined number of pixels that form the target region is compared to a predetermined threshold value which is known to be suitable for infant sleep monitoring. If the number of pixels is less than the threshold value, the image is determined to be unsuitable; if the number of pixels is greater than the threshold value, the image is determined to be suitable if the orientation of the image is also deemed to be suitable. The predetermined threshold size in this exemplary embodiment is between 550,000 and 950,000 pixels. In this exemplary embodiment, the image is only deemed to be suitable if the size of the target region is greater than the predetermined threshold size and the orientation of the target region is landscape.

If it is determined in the step 130 that the image is unsuitable, the method proceeds to a step 140 in which instructions for adjusting the image sensor are provided. In this exemplary embodiment, if the image is determined to be unsuitable for use in monitoring the sleep of the infant, the instructions provided in the step 140 include instructions as to how to adjust the position of the image sensor to ensure accurate infant monitoring (e.g., the instructions may be to reduce the height of the image sensor above the bed/crib and move the image sensor to the long side of the bed/crib). These instructions may be provided to a user via a user interface or to an automated image sensor adjustment system.

In detail, in this exemplary embodiment, if the area of the target region is found to be less than the predetermined threshold size, instructions are provided to a user to reduce the vertical height of the image sensor so that the image sensor is closer to the position where the infant will lie. If the orientation of the target region is found to be portrait, then instructions are provided to a user via a user interface to move the image sensor to the long side of the infant's crib so that the target region is in a landscape orientation. If the area of the target region is greater than the predetermined threshold size, and the orientation of the target region is landscape then a notification is provided to the user that the image sensor is in the correct position.

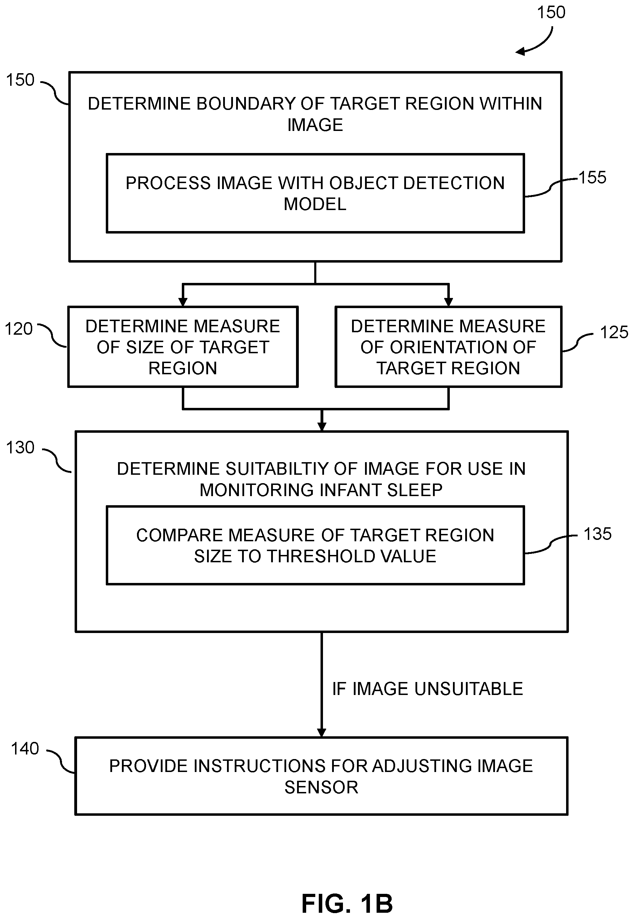

Referring now to Fig. 1B there is depicted a simplified flow diagram of a computer-implemented method 150 for assessing the suitability of an image for use in monitoring an infant according to another embodiment. Again, the method will be described with reference to the preferred implementation of sleep monitoring.

The method 150 is similar to the method 100 but commences with a step 150 different from the step 110 of method 100. Like numerals are used to denote steps in Fig. 1B identical to those in Fig. 1A and a discussion of these steps will not be repeated.

The step 150 comprises determining a boundary of a target region within the image, wherein the target region is a region of the image to be used for monitoring the sleep of the infant. In this exemplary method, the step 150 further comprises the sub-step 155 of processing the image with an object detection model to identify the boundary of the target region. For example, a trained machine learning model may be used to detect the mattress area of an infant's crib within an image of the infant's nursery. The trained model outputs the bounding box of the detected mattress which is used as the boundary of the target region.

Although an object detection model is used in this exemplary embodiment, in other embodiments a different model may be employed to determine the target region within the image data. Any suitable model may be used including but not limited to an object detection model, an edge detection model, and a contrast enhancement model.

Although in the methods 100 and 150, the measure of the size of the image is the number of pixels that make up the target region, other suitable measures of the size of the image may be used in different embodiments. For example, the measure of the size of the image may comprise at least one of: an area of the target region; a length of the target region; and the proportion of the image covered by the target area. The target region may cover any proportion of the image up to and including the entirety of the image.

Similarly, other aspects of the methods 100 and 150 may be implemented differently in alternative embodiments. For instance, the target region need not be rectangular and therefore the measure of the orientation of the target region may not be simply portrait or landscape. In some embodiments, the measure of the orientation of the target region may be a value describing the angle between a certain axis of the target region and an axis of the image.

The suitability assessment need not rely on both the orientation and the size of the target region but may depend on only one of these values i.e. in alternative embodiments only one of the steps 120 and 125 may be included. In some instances, it may only be possible to obtain one of the size and orientation of the target region, for example the orientation may not be possible to calculate if the target region has a particularly irregular shape, and therefore only one of these measures may be used. However, using both the orientation and the size of the target region may result in more accurate determination of the image's suitability.

In some embodiments, the determination of image suitability may further involve comparing the size of the target region to a maximum threshold value, and only determining the image is suitable if the size of the target region is less than this maximum threshold value. A maximum suitable size of the target region may help to reduce the processing power required for monitoring the infant.

Methods 100 and 150 result in a binary determination that either the image is suitable, or it is unsuitable. In alternative embodiments, a more nuanced suitability assessment may be provided. For example, the suitability may be classified by a quantitative or qualitative value that ranks the suitability of the image on some predetermined scale (e.g., with a rank out of 5). This may allow for more detailed feedback to be provided on how much the image sensor set-up needs to be adjusted to allow for accurate infant monitoring.

The predetermined threshold size value may be adjusted depending on the resolution of the image sensor being used, the level of background noise in the image data, the real size of the infant's bed/crib, or the age of the infant being monitored (older babies may have larger features and movements and therefore accurate monitoring of older infants may have less strict requirements on image resolution). Therefore, the suitability assessment of the present invention in some embodiments may rely on further information provided by a user or by the image sensor.

In the methods 100 and 150, the step 140 comprises providing an instruction to adjust the position of the image sensor used to capture the image. However, in alternative embodiments, the instructions may comprise at least one of: an instruction to adjust the orientation of the image sensor; an instruction to adjust the operating parameters of the image sensor; and an instruction to adjust the optical zoom of the image sensor. In some envisioned embodiments, a more general output relating to the suitability of the image for use in infant monitoring is provided. For example, a value or description of the suitability of the image may be output without providing specific instructions for adjusting the parameters of the image sensor. This may be advantageous in allowing flexibility in applying the proposed method to different image sensor and infant monitoring system set-ups which may require different levels and types of adjustment.

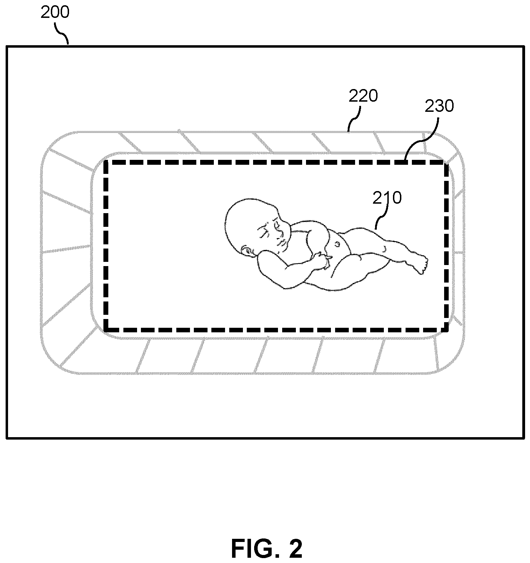

Referring now to Fig. 2, there is illustrated an image 200 containing a target region 230 to be used for monitoring an infant, for example the sleep of an infant, according to a proposed embodiment.

The image 200 depicts an infant 210 within a crib 220. The boundary of a target region 230 is indicated by the dashed box. This target region corresponds to the image data of the image that is to be used for monitoring the sleep of the infant, in this instance the mattress area of the crib 220. The image data of the target region may be intended to be used for sleep monitoring functions including at least one of: monitoring the breathing of the infant; monitoring the movement of the infant; monitoring the vital signs of the infant; monitoring the presence of the infant in the monitored area; and sleep stage tracking of the infant.

Although depicted as a rectangular region, in other images the target region may be of a different shape, including but not limited to: a plurality of rectangular regions; a circular shape; and an irregular shape. The boundary of the target region 230 may be determined automatically (e.g., using a trained machine learning model) or from data received from a user who may select the boundary of the target region using a touch-sensitive screen.

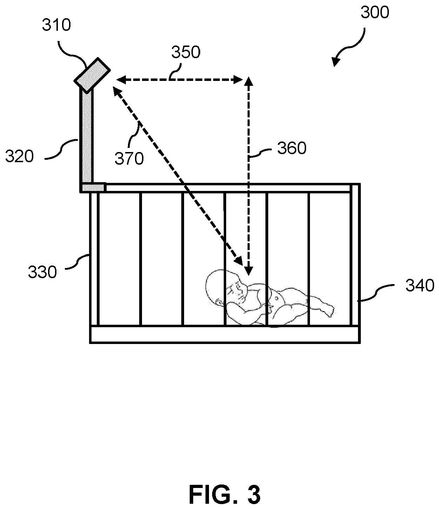

Referring now to Fig. 3 there is depicted an infant monitoring system set-up 300 according to a proposed embodiment.

An image sensor 310 is attached via a mount 320 to an infant's crib 330. The image sensor is configured by this set-up to obtain bird's-eye images of the mattress area of the crib 330. For example, the image sensor 310 may be used to capture the image 200.

The mount 320 is configured such that the height of the image sensor above the base of the crib 300 can be adjusted. Further, the position of mount along the side of the crib is also adjustable.

Once an image has been captured with the image sensor 310, it is processed using any of the above described computer-implemented methods for assessing the suitability of an image for use in monitoring the sleep of an infant which rely on the use of a measure of the size and/or orientation of a target region of the image. The size of the target region in the image is a useful metric for the vertical height 360 of the image sensor above the infant's sleeping position, as the dimensions of the target region will decrease as the height of the image sensor increases. Thus, the image analysis may enable an indication, if not a direct measurement, of a distance between the image sensor and the infant. An object detection model may for example be used to provide an indication of distance.
The orientation of the target region may be a useful metric for the horizontal distance 350 between the image sensor and the sleeping position of the crib (if the image sensor is positioned along the long edge of the infant's crib the infant is more likely to be closer to the image sensor than if the image sensor is positioned along the short edge of the crib which may be the opposite end of the crib to the sleeping infant). Using these two measures (size and orientation) in conjunction with one another, allows for information to be gathered about the straight-line distance 370 between the image sensor and the infant.

All three distances 350, 360, and 370 will affect the quality of an image taken by the image sensor 310. To accurately monitor the infant's features and movements using the image sensor 310, the image sensor must be sufficiently close to the position of the infant such that the infant is imaged with sufficient resolution.

Once the suitability of the image has been assessed by any of the proposed methods of the present invention, if the image is determined to be unsuitable, instructions are provided to a user via a separate user interface device (not shown) for adjustments to be made to the image sensor 310. Possible adjustments include adjusting the height of the image sensor above the crib, adjusting the orientation of the image sensor 310 or adjusting the position of the image sensor along the side of the crib. If the image sensor 310 is equipped with optical zoom capabilities, the zoom of the image sensor may also be adjusted to ensure the infant is depicted in a large enough proportion of the field of view.

In the image monitoring set-up 300 adjustments to the image sensor 310 are achieved manually by a user. However, in other proposed embodiments, the image sensor 310 and the mount 320 may be adapted to allow for automatic adjustment of the position and orientation of the image sensor. This may involve continual automatic position and orientation adjustment of the image sensor throughout an infant sleep period to allow for dynamic monitoring of the infant. This may ensure that images captured by the image sensor are suitable even if the infant changes its position.

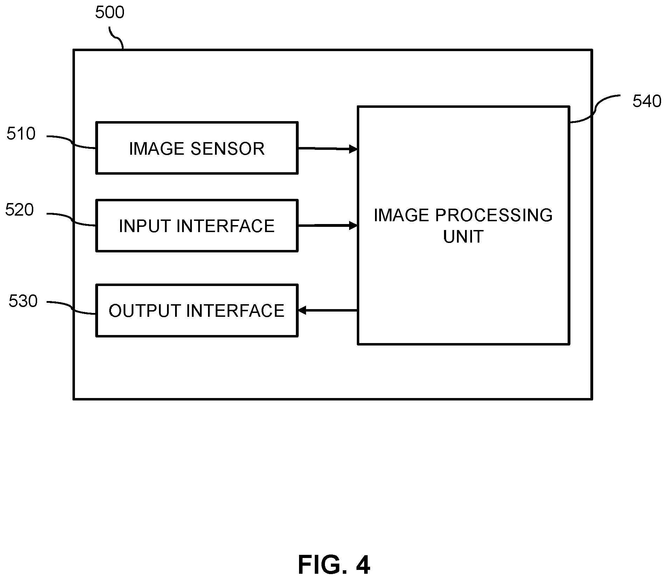

Referring now to Fig. 4, there is depicted a simplified block diagram of an infant monitoring system 400 for assessing the suitability of an image of a monitored area for monitoring an infant, for example for monitoring the sleep of an infant.

The infant monitoring system 400 comprises an image sensor 410, an input interface 420, output interface 430 and image processing unit 440.

The system 400 is configured to assess the suitability of an image, captured by the image sensor 410, of a monitored area for monitoring the sleep of an infant. It achieves this by processing the image from the image sensor 410 in the image processing unit. This may be carried out using the method 100.

In detail, the image sensor 410 produces image data of a monitored area. The input interface 420 permits a user to select a target region of the image data that corresponds to a region of the image that will be used for monitoring the infant. The processing arrangement 440 receives the image form the image sensor 410 and an indication of the boundary of the target region from the input interface 420.

The image processing unit 440 is configured to run a computer program comprising code means for implementing the method 100. Thus, on receiving the image from the image sensor and the indication of the boundary of the image from the input interface 420, the processor uses a computer implemented suitability assessment method to determine the size of the target region and from this determine the suitability of the image for use in monitoring the sleep of the infant. The image processing unit is further configured to output to the output interface 430 a signal describing the suitability of the image, and if the image is determined to be unsuitable, instructions to a user for adjusting the image sensor so as to improve the suitability of future images.

The output interface 430 outputs the suitability assessment along with any instructions for adjustments to the image sensor to a user. This may be achieved via an electronic output signal and/or a visible/audible output.

Although in the system 400, the image sensor 410, the input interface 420, and the output interface 430 are present in the same device, it will be understood that these components may be distributed among various devices. For example, there may be provided an infant monitor comprising only an image sensor. The image data taken by the image sensor of the image monitor may then be relayed to a personal smart device of the parent/care. The processing and interface capabilities of the personal smart device may fulfil the functionalities of the input interface 420, image processing unit 440, and the output interface 430.

In an alternative embodiment, an image monitoring system may comprise more than one image sensor. Each image sensor may have different set-up parameters (e.g., different lens set-ups, sensors with different resolution, or different positions). In this case, the instructions for adjusting the image sensor may comprise instructions to change which image sensor of the image monitoring system is used to monitor the sleep of the infant. This embodiment may allow for a greater variety of possible image set-ups and therefore an increased likelihood that suitable images of the infant may be captured.

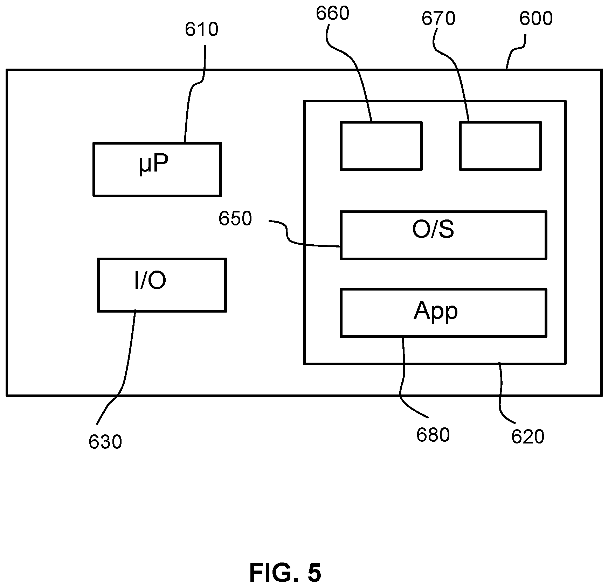

Fig. 5 illustrates an example of a computer 500 within which one or more parts of an embodiment may be employed. Various operation discussed above may utilize the capabilities of the computer 500. In this regard, it is to be understood that system functional blocks can run on a single computer or may be distributed over several computers and locations (e.g. connected via internet).

The computer 500 includes but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor-based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules om accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 530 may include input devices such as, for example but not limited to, a mouse, keyboard, scammer, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this documents a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figures 1A, and 1B, and the system of Fig. 5, may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer- readable storage medium stores a computer program comprising computer program code configured to cause one or more physical computing devices to carry out a method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more program stored thereon can be loaded into a processor.

To the extent that an embodiment is implemented partly or wholly in hardware, the blocks shown in the block diagrams of Fig. 5 may be separate physical components, or logical subdivision of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrate circuits (ASICs) and field-programmable gate arrays (FPGAs). One or more blocks may be implemented as a combination of dedicated hardware to perform some functions and one or more programmed microprocessors and associated circuity to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measure cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or descriptions, it is noted the term "adapted to" is intended to be equivalent to the tram "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations the functions noted in the block may occur out of the order noted in the figures. For example, two blocks shown in succession may, in fact be executed substantially concurrently, or the blocks may sometimes be executed in the reverse order, depending on the functionality involved. It will also be noted that each block of the block diagrams and/or flow diagrams, and combinations of blocks in the block diagrams and/or flow diagrams, can be implemented by special purpose hardware-based systems that perform the specified functions or acts or carry out combinations of special purpose hardware and computer instructions the architecture, functionality, and operation of possible implementations of systems, methods, and computer program products according to various embodiments of the present invention.

## Claims

1. A computer-implemented method for assessing the suitability of an image, captured by an image sensor, of a monitored area for use in monitoring an infant, the method comprising:
determining a boundary of a target region within the image, or receiving an indication of a boundary of a target region within the image, wherein the target region is a region containing image data to be used for monitoring the infant;
determining a measure of at least one of: a size; and an orientation of the target region of the image;
determining, from the determined measure of the size and/or orientation of the target region, a suitability of the image for use in monitoring the infant; and
providing an output based on the determined suitability of the image.

2. The method of claim 1, wherein the method determines a measure of the size of the target region and determining a suitability of the image for use in monitoring the infant further comprises:
determining the image to be suitable if the measure of the size of the target region is greater than a predetermined threshold size; and
determining the image to be unsuitable if the measure of the size of the target region is less than the predetermined threshold size.

3. The method of claim 2 wherein the measure of the size of the target region comprises the area of the target region in pixels and the predetermined threshold size is between 550,000 and 950,000 pixels.

4. The method of any preceding claim, wherein the measure of the size of the target region comprises at least one of: an area of the target region; a dimension of the target region; and the proportion of the image covered by the target area.

5. The method of any preceding claim, wherein the measure of the orientation of the target region comprises at least one of: portrait; landscape; and an angle between an axis of the target region and an axis of the image.

6. The method of any preceding claim, wherein determining a boundary of a target region within the image comprises processing the image with at least one of: an object detection model; an edge detection model; and a contrast enhancement model.

7. The method of any preceding claim, wherein receiving an indication of a boundary of a target region within the image comprises receiving data provided by a user using a user interface.

8. The method of any preceding claim, wherein providing an output regarding the suitability of the image comprises providing instructions for adjusting the image sensor when the image is determined not to be suitable.

9. The method of claim 8, wherein providing instructions for adjusting the image sensor when the image is determined not to be suitable comprises at least one of:
providing an instruction to adjust the position of the image sensor;
providing an instruction to adjust the orientation of the image sensor;
providing an instruction to adjust the operating parameters of the image sensor;
providing an instruction to adjust the optical zoom of the image sensor; and
providing an instruction to use another image sensor.

10. The method of any preceding claim, wherein monitoring the infant comprises at least one of:
monitoring the infant's breathing;
monitoring the infant's vital signs;
monitoring the infant's movement;
monitoring the infant's position; and
tracking the infant's sleep stages.

11. A computer program comprising code means for implementing the method of any preceding claim when said program is run on a processing system.

12. A processing arrangement comprising the computer program of claim 11 and configured to execute the computer program,

13. A system for assessing the suitability of an image, captured by an image sensor, of an infant in a monitored area for use in monitoring the breathing of the infant, the system comprising:
a processing arrangement configured to run the computer program of claim 11.

14. An infant monitoring system comprising:
the system of claim 13; and
an image sensor configured to capture the image of the infant in the monitored area and optionally wherein the processing arrangement is further configured to automatically adjust the image sensor.

15. The system of any of claims 13 and 14, further comprising at least one of:
a user input interface configured to permit the user to select the boundary of the target region within the image; and
an output interface configured to:
provide to the user an indication of the suitability of the image for use in monitoring the breathing of the infant; and
provide instructions to the user for adjusting the image sensor when the image is determined not to be suitable.
